Europäisches Patentamt

European Patent Office

Office européen des brevets

(11) Publication number: **0 037 740**
**B1**

(12) **EUROPEAN PATENT SPECIFICATION**

(45) Date of publication of patent specification: **21.11.85**

(21) Application number: **81301521.1**

(22) Date of filing: **07.04.81**

(51) Int. Cl.⁴: **A 61 K 9/50,** A 61 K 9/52,
A 61 K 9/42

(54) **Solid microdose drug preparation.**

(30) Priority: **07.04.80 JP 46002/80**

(43) Date of publication of application:
**14.10.81 Bulletin 81/41**

(45) Publication of the grant of the patent:
**21.11.85 Bulletin 85/47**

(84) Designated Contracting States:
**CH DE FR GB IT LI**

(56) References cited:
GB-A-1 415 256
US-A-4 102 806
US-A-4 132 753

CHEMICAL ABSTRACTS, vol. 89, 1978, page
372, abstract 12147d, COLUMBUS, OHIO (US)
CHEMICAL ABSTRACTS, vol. 93, 1980, page 85,
abstract 19440k, COLUMBUS, OHIO (US) T.
SEKI et al.: "Effects of a new bronchodilator,
3-formamido-4-hydroxy-alpha-(N-(p-methoxy-
alpha-methyl-phenethyl)amino)-methyl)benzyl
alcohol fumarate dihydrate on healthy
subjects)

(73) Proprietor: **YAMANOUCHI PHARMACEUTICAL
CO. LTD.
No. 5-1 Nihonbashi-Honcho, 2-chome Chuo-ku
Tokyo (JP)**

(72) Inventor: **Fukui, Muneo
No.3184-15, Oyaguchi
Urawa-shi Saitama (JP)**
Inventor: **Kubota, Yukio
No.1-11-2, Tagara
Nerima-ku Tokyo (JP)**
Inventor: **Kawata Hiroitsu
No.1-7, Namiki
Kawagoe-shi Saitama (JP)**
Inventor: **Konno, Yutaka
No.21-6, Besshocho
Omiya-shi Saitama (JP)**
Inventor: **Aruga, Masayoshi
No. 5-16-14, Midorigaoka
Ageo-shi Saitama (JP)**

(74) Representative: **Geering, Keith Edwin et al
REDDIE & GROSE 16 Theobalds Road
London WC1X 8PL (GB)**

Courier Press, Leamington Spa, England.

(56) References cited:
**CHEMICAL ABSTRACTS, vol. 85, 1976, page
342, abstract 83189x, COLUMBUS, OHIO (US)
B.F. JOHNSON et al.: "Comparative
pharmacokinetics of beta-methyl digoxin and
digoxin administered as tablets or
encapsulated solution"**

# 0 037 740

**Description**

This invention relates to solid preparations of microdose drugs.

Obtaining content uniformity in solid drug preparations is no problem for preparations having a large content of drug per unit of the preparation, i.e. a large content of drug per tablet, capsule or unit dose of the package, but it is a serious problem in case of microdose drug preparations having a drug content of less than 50 mg, particularly less than few milligrams. Heretofore, microdose drug preparations have been obtained by a solution method wherein a solution of a drug is uniformly dispersed in excipient and the dispersion is formed into a preparation, or by a powder dilution method wherein a drug is compounded with excipient in the form of a diluent powder and the resulting product is formed into the preparation.

In the powder dilution method it is frequently difficult to mix the drug uniformly with excipient and hence to obtain uniformity of drug content so that complex manufacturing procedures (e.g. a homogenizing step such as mixture pulverization), are required. The solution method may be superior to the powder dilution method as regards giving uniformity of drug content, but the solution method is liable to other difficulties—e.g. since the drug is dissolved during the procedure, its crystal form may change, for example, a hydrate may change into a solvate to change the drug itself; and when the solution is dispersed in excipient its particle size becomes very small, so that the stability of the drug to the passage of time is low. It is very important for preparations of extremely low dose drugs (e.g. having a drug content of less than 1 mg per unit of preparation) to assure drug content uniformity and the stability of the effective component. Since the extremely low dose drugs generally have strong medicinal activity, if the uniformity of drug content and the stability of the effective component are not strictly assured, there is a possibility that the desired medicinal effect will not be obtained or that unforeseen accident by overdose may occur.

The present invention provides a solid preparation of microdose drug in which the drug is present as wax-coated particles.

Solid drug preparations according to the invention having good drug content uniformity can be obtained even by simple manufacturing procedures, e.g. by the direct compression method. With the use of wax coating even aggregates of drugs can be fixed in well-dispersed form. Due to the wax coating, the surface property of the drug can be masked and hence the loss of the drug by electrostatic or adherent attachment of the drug can be prevented; heretofore even if the absolute amount of loss in this way has been small, the influence of the loss expressed as a percentage of the calculated amount is large and cannot be overlooked in the case of the microdose preparations.

A microdose preparation in this invention means an extremely low dose preparation which contains less than 1 mg, of active component per unit of the preparation. The drugs suitable for use in this invention are β-methyldigoxin, and formoterol fumarate. Examples of suitable waxes are fats and oils prepared by hydrogenating vegetable oils such as sesame oil, olive oil, soybean oil, corn oil, coconut oil and castor oil, (e.g., hardened oils such as those known under the trade names—"Lubriwax 101" and "Lubriwax 102H") made by Freund Industrial Co., Ltd.—and "Himako"—hardened castor oil made by Kawaken Fine Chemical Co., Ltd.; waxes such as beeswax, carnauba wax, spermaceti; hydrocarbons such as paraffin or ceresin; fatty acids such as myristic acid, palmitic acid or stearic acid; higher alcohols such as palmitic alcohol or stearyl alcohol; polyhydric alcohols such as PEG (macgrogol) or batyl alcohol; and esters such as octadecyl palmitate or octadecyl stearate.

The particles of microdose drug coated with wax may be a powder of a microdose drug coated with wax directly or together with a definite amount of excipient, of a powder product prepared by dispersing a liquid microdose drug into a wax directly or after dispersing the liquid microdose drug in a definite amount of excipient. Coating of a powder of a microdose drug with wax directly or together with a definite amount of excipient is performed by uniformly dispersing the powder into molten wax or by uniformly dispersing the powder into wax dissolved in or mixed with appropriate solvent and then removing the solvent, e.g. by vacuum drying or spray drying. In this dispersing procedure, microdose drug powder of particle size less than 100 μm is usually used. After uniformly dispersing a liquid microdose drug into wax directly or after dispersing the drug in a definite amount of excipient, the drug coated with the wax is formed into powder or granules—of course, when solvent removal is effected by e.g. spray drying, a separate powdering or granulating step is not necessary.

The proportion of wax used is preferably 0.05—5 times, most preferably 0.3—2 times the amount of the microdose drug. As diluting solvent, one or more ordinary organic solvents (e.g. selected from methylene chloride, chloroform, carbon tetrachloride, ether, benzene, acetone, tetrahydrofuran and ethyl acetate) which do not affect the microdose drug are suitably used, but solvents which do not dissolve the drug itself are preferred. There is no particular restriction of the amount of solvent used but the smallest amount thereof necessary for dissolving the wax or for mixing with the wax is suitable.

A small amount of surface active agent etc. may be used for uniformly dispersing a microdose drug powder into wax or increasing the adhesion between the microdose drug and wax. Materials suitable for the purpose include hydroxypropylmethyl cellulose (TC-5), polyvinylpyrrolidone (PVP), β-cyclodextrin, and nonionic surface active agents (e.g. sorbitan fatty acid esters, polyoxyethylene sorbitan fatty acid esters, polyoxyethylene fatty acid esters, polyoxyethylene higher alcohol ethers, glycerol fatty acid esters and polyoxyethylene castor oil derivatives). For increasing the photostability of the microdose drug, a light shielding agent such as titanium dioxide may be incorporated.

3

# 0 037 740

Powdering or granulation of the drug coated with wax can be performed by dispersing the drug into wax mechanically or by an ultrasonic treatment under heating, if necessary (with molten wax when directly dispersing the drug in the wax), and thereafter spray congealing, vacuum drying, or spray drying the dispersion.

A solid drug preparation of this invention is obtained by formulating the wax-coated powder of the drug. The preparation can be formulated in an ordinary manner. For example, when the drug coated with wax is formulated by a simple conventional preparation step such as a direct compression method, it disperses well and a preparation with excellent uniformity of the effective component can be obtained.

This effect is particularly remarkable in the case of preparations of microdose drugs which have strong aggregation properties. Another feature of the preparations of this invention is the good stability of the effective component; reduction or alteration of the crystallinity of the microdose drugs, and interaction between excipient and microdose drug can be prevented by the wax coating to improve the drug stability of the preparations during formation and on storage.

The dissolution properties of drugs coated with hydrophobic waxes are generally poor. The dissolution properties of drugs are influenced by the kind and amount of waxes used and also by the kind of treating solvents employed, and hence investigation of the physicochemical properties of drugs and waxes together is necessary. When the dissolution properties of drug coated with hydrophobic waxes are poor, hydrophilic excipients may be used in the preparations to improve these properties.

The following test results illustrate the properties of solid drug preparations according to the invention.

I. Drug content uniformity

Tests of the content uniformity of formoterol fumarate and $\beta$-methyldigoxin in tablet preparations according to the invention were performed by the test method shown below and the results are shown in Table I.

TABLE I

| Example | Mean content % (n=20) | Standard deviation % | Coefficient of variation % | Max. % Min. % |
|---|---|---|---|---|
| 1 | 100.2 | 2.93 | 2.92 | 104.4 92.5 |
| 2 | 99.5 | 1.87 | 1.88 | 103.5 96.6 |
| 3 | 97.5 | 2.19 | 2.25 | 102.7 94.8 |
| 4 | 96.1 | 1.61 | 1.68 | 99.6 92.3 |
| 5 | 98.9 | 1.57 | 1.59 | 101.6 96.0 |
| 6 | 97.3 | 2.98 | 3.06 | 101.8 88.7 |
| 7 | 95.5 | 2.08 | 2.18 | 100.8 93.4 |
| 8 | 99.7 | 2.43 | 2.44 | 104.4 94.4 |
| 9 | 94.2 | 1.79 | 1.90 | 98.2 91.1 |
| 12 | 98.4 | 3.67 | 3.73 | 109.8 93.9 |
| 13 | 95.5 | 1.78 | 1.87 | 99.6 89.3 |

As shown in Table I the coefficient of variation for formoterol fumarate and $\beta$-methyldigoxin was less than 4%, showing the good content uniformity.

4

Test methods:

(a) The content uniformity of the formoterol fumarate preparation (tablet) was tested by the following method.

To each table containing formoterol fumarate (20 µg/tablet) was accurately added 10 ml of an aqueous solution of 0.2% sodium chloride and after heating to about 50°C for 10 minutes, the mixture was shaken for 15 minutes. Then the mixture was subjected to centrifugal separation and the drug content of a fixed volume of the supernatant was determined by high-performance liquid chromatography on a column of Lichrosorb RP-18 (trade name) made by Merck & Co., Inc.

(b) The content uniformity of the β-methyldigoxin preparation (tablet) was tested as follows.

Each tablet of β-methyldigoxin preparation (100 µg/tablet) was placed in a 50 milliliter flask and after adding thereto 2.5 ml of water and allowing to stand for 10 min to disintegrate the tablet, 40 ml of absolute ethanol was added. The mixture was immersed in an ultrasonicator for 10 min (50 KHz, 150 Watts) and then absolute alcohol was added thereto to make up to 50 ml accurately. The solution was subjected to centrifugal separation and the drug content of 1 ml of the supernatant obtained was determined according to the fluorometeric assay method described in "Yakuzai Gaku (Pharmacology)"; *37*, 215 (1977) by Sonobe et al.

II. Stability:

The stability of formoterol fumarate (in tablet preparations according to the invention) to the passage of time was tested by the following test method. The results are shown in Table II.

TABLE II:
Stability of formoterol fumarate

| | Wax | Amount** | Solvent | Production method | % Remaining after storage closed at 60°C for 3 months |
|---|---|---|---|---|---|
| Control* | — | — | — | (A) | 52.2 |
| (untreated) | — | — | — | (B) | 63.8 |
| Example 10 | Castor wax | 50 | Acetone | (B) | 82.8 |
| Example 11 | Castor wax | 50 | Dichloromethane | (B) | 85.1 |

* Control: Produced by the general method described in Examples 1—11 shown below.
** Amount: Weight ratio to 100 of a microdose drug.
(A): Wet granulation method.
(B): Direct compression method.

As shown in Table II, the formoterol fumarate is markedly stabilized by the wax coating of the drug.

Test method:

The stability of the formoterol fumarate preparation (tablet) to the passage of time was tested by the following method.

The weight of 10 tablets of formoterol fumarate preparation (20 µg/tablet) was accurately measured and after adding 18 ml of an aqueous solution of 0.2% sodium chloride and heating the mixture to about 50°C for 10 min, the mixture was shaken for 15 min and filtered by means of a glass filter. The residue was washed with an aqueous solution of 0.2% sodium chloride and filtered. The whole filtrates were combined with each other to make up to exactly 25 ml and the drug determined in the same manner as in the uniformity test described above. The results obtained before and after storage in a closed container at 60°C for 3 months were compared to give the values in the final column of Table II.

III. Loss of microdose drugs during mixing procedures:

Test results for the loss of formoterol fumarate during mixing when using the wax-coated powder thereof and in the case of non-coated powder thereof are shown in Table III.

TABLE III

| | Wax coating | |
| Number of test | None (control) | Coated |
| --- | --- | --- |
| 1 | 12.0 | 1.9 |
| 2 | 10.3 | 5.3 |
| 3 | 14.9 | 2.5 |

Working conditions: 18—24°C, 40—27% RH.

Test method:

The wax-coated powder of formoterol fumarate and the non-coated powder of formoterol fumarate obtained in Example 10(a) below were separately mixed with crystalline lactose, and pulverized into particles finer than 80 mesh to form respective diluted powders which were sieved to pass particles finer than 80 mesh. Then each particle product was mixed separately with crystalline lactose, crystalline cellulose (Avicel PH 102: a trade name, made by Asahi Chemicals Co., Ltd.) and potato starch using a V-type mixer and then the percentage losses of formoterol fumarate during the respective mixing procedures were measured. When the wax-coated powders were used, adhesion of formoterol fumarate to the inside wall of the mixer did not occur.

Solid drug preparations according to this invention and their production, are illustrated by the following Examples.

Examples 1—11

The solid drug preparations of this invention were produced by the following general methods.

(a) Production of wax-coated powder:

A fixed amount of wax was dissolved in 10 ml of a solvent and after suspending 1.5 g of microdose drug in the solution, the suspension was stirred by hand. Then, after evaporating the solvent by means of a rotary evaporator, the residue was vacuum-dried for 4 hours at room temperature using phosphorus pentoxide. Then the residue was sieved to pass the powders through 150 mesh using a brush, whereby wax-coated powder of the microdose drug was obtained.

(b) Production of solid drug preparation (tablet):

(i) Direct compression method

Wax-coated powder of microdose drug obtained in step (a) was mixed with crystalline lactose pulverized into particles below 80 mesh to form diluted powder and then the diluted powder was sieved to pass the particles below 80 mesh. The particles were mixed with crystalline lactose, crystalline cellulose (Avicel PH 102, a trade name, made by Asahi Chemicals Co., Ltd.) and potato starch using a V-type mixer for 10 minutes and after adding thereto a lubricant, the mixture was formed into tablets of 6 mm diameter and 75 mg/tablet. Each tablet contained 20 μg of the microdose drug.

(ii) Wet granulation method

Wax-coated powder of microdose drug obtained in step (a) was mixed with crystalline lactose pulverized into particles below 80 mesh to form a diluted powder and then the diluted powder was mixed with pulverized crystalline lactose, crystalline cellulose (Avicel PH 101), and potato starch and a fluidized bed granulation was performed using the potato starch paste as a binder. The particles formed were dried for 30 minutes at 50°C and sieved to pass particles through 32 mesh. Then the particles were compounded with a lubricant and formed into tablets by the method of step (i) described above. Each tablet contained 20 μg of the microdose drug.

Practical Examples of this invention are shown in the following Table III.

TABLE IV

| Example | Microdose drug | (a) Production of wax-coated powder | | | (b) Production of solid drug preparation (tablet) |
|---|---|---|---|---|---|
| | | Wax | Amount* | Solvent | |
| 1 | Formoterol fumarate | Lubriwax 102 H | 5 | Dichloromethane | (B) |
| 2 | " | " | 5 | " | (A) |
| 3 | " | " | 30 | " | (B) |
| 4 | " | " | 30 | " | (A) |
| 5 | " | " | 200 | " | (B) |
| 6 | " | Lubriwax 101 | 100 | Acetone | (B) |
| 7 | " | " | 100 | " | (A) |
| 8 | " | Castor wax | 100 | " | (B) |
| 9 | " | " | 100 | " | (A) |
| 10 | " | " | 50 | " | (B) |
| 11 | " | " | 50 | Dichloromethane | (B) |

Amount*: Weight ratio to 100 of the microdose drug.
(A): Wet granulation method;
(B): Direct compression method.

Example 12

(a) Production of wax-coated powder of β-methyldigoxin:

In a 100 milliliter eggplant type flask was placed 1.5 g of Lubriwax 102H (trade name, made by Freund Industrial Co., Ltd.) and the wax was dissolved in 50 ml of a carbon tetrachloride solution of 0.5% polyoxyethylene castor oil derivative HCO-60 (trade name, made by Nikko Chemicals Co., Ltd.) under heating. In the solution was suspended 1.5 g of β-methyldigoxin and the flask was immersed in an ultrasonicator for 5 min at 50 KHz and 150 watts, whereby a good suspension was obtained. (Note: When HCO-60 was not added, extensive aggregation of β-methyldigoxin occurred). The suspension was distilled by means of a rotary evaporator to remove the solvent and after drying the residue for 4 hours under reduced pressure at room temperature using phosphorus pentoxide, the product was sieved to pass particles through 150 mesh, whereby the wax-treated product of β-methyldigoxin was obtained.

(b) Production of β-methyldigoxin tablets by direct compression method:

The wax-treated product of β-methyldigoxin obtained in step (a) described above was mixed with crystalline lactose pulverized into particles below 80 mesh to form a diluted powder and the diluted powder was sieved to pass particles through 80 mesh. The particles were then mixed with crystalline lactose, crystalline cellulose (Avicel PH 102), and corn starch by means of a V-type mixer and after adding lubricant thereto lubricant, the mixture was formed into tablets of 7 mm diameter and 120 mg/tablet. Each tablet contained 100 μg of β-methyldigoxin.

Example 13

(a) Wax-coated powder of β-methyldigoxin was produced in the same manner as in Example 12(a) described above.

(b) Production of β-methyldigoxin tablets by wet granulation method:

The wax-treated powder of β-methyldigoxin obtained in step (a) described above was mixed with crystalline lactose pulverized to particles below 80 mesh to form a diluted powder and then the diluted powder was subjected to fluidized bed granulation using 2% potato starch paste as a binder. The granules obtained were dried for 30 min at 50°C, sieved to pass particles through 32 mesh, and after mixing with a lubricant, the mixture was formed into tablets in the manner of Example 12(b). Each tablet contained 100 μg of β-methyldigoxin.

**0 037 740**

**Claims**

1. A solid preparation of microdose drug selected from formoterol fumarate and β-methyldigoxin characterised in that it contains wax-coated particles of said drug and in that the content of said drug is less than 1 mg per unit of the preparation.

2. A preparation according to claim 1, characterised in that the proportion of wax in the wax-coated drug is 0.05—5 parts by weight per 1 part by weight of said drug.

3. A preparation according to claim 1 or 2 characterised in that the wax comprises hydrogenated oil.

4. A method of producing a solid preparation of microdose drug selected from formoterol fumarate and β-methyldigoxin characterised by first forming wax-coated particles of said drug by (1) dispersing said drug directly in molten wax, or (2) dispersing said microdose drug in wax dissolved in a solvent and removing the solvent and forming powder or granules of the resultant dispersion or residue by spray congealing, vacuum drying or spray drying.

5. A method according to claim 4, characterised in that the microdose drug together with excipient is coated with the wax.

6. A method according to claim 4 or 5, characterised in that the proportion of wax coating is 0.3—2 parts by weight per one part by weight of said drug.

**Patentansprüche**

1. Feste Verabreichungsform zur Mikrodosierung eines Arzneimittles in Form von Formoterolfumerat und/oder β-Methyldigoxin dadurch gekennzeichnet, daß die Arzneimittelteilchens mit Wachs überzogen sind und der Arzneimittelgehalt der Verabreichungsform je Einheit <1 mg beträgt.

2. Verabreichungsform nach Anspruch 1 dadurch gekennzeichnet, daß der Anteil von Wachs auf den Arzneimittelteilchen 0,05 bis 5 Gewichtsteile je Gewichtsteil Arzneimittel beträgt.

3. Verabreichungsform nach einem der Ansprüche 1 oder 2 dadurch gekennzeichnet, daß das Wachs ein hydriertes Öl enthält.

4. Verfahren zur Herstellung der Verabreichungsform zur Mikrodosierung eines Arzneimittels in Form von Formoterolfumarat und/oder β-Methyldigoxin dadurch gekennzeichnet, daß man zuerst die mit Wachs überzogenen Teilchen des Arzneimittels hergestellt durch (1) Dispergieren des Arzneimittels in geschmolzenem Wachs oder (2) Dispergieren der Arzneimittel-Mikrodosis in Wachs, welches in einem Lösungsmittel gelöst ist, anschließend Entfernen des Lösungsmittels und Bildung eines Pulvers oder Granulats aus der Dispersion oder dem Rückstand durch Sprüh-Erstarrung, Vakuumtrocknung oder Sprühtrocknung.

5. Verfahren nach Anspruch 4, dadurch gekennzeichnet, daß man die Arzneimittel-Mikrodosis zusammen mit Arzneimittelträger mit Wachs überzieht.

6. Verfahren nach einem der Ansprüche 4 oder 5, dadurch gekennzeichnet, daß der Wachsanteil 0,3 bis 2 Gewichtsteile je Gewichtsteil Arzneimittel beträgt.

**Revendications**

1. Composition solide de médicament en microdose choisi entre le fumarate de formotérol et la β-méthyldigoxine, caractérisée par le fait qu'elle contient des particules de médicament solide revêtues de cire et que la teneur en médicament est inférieure à 1 mg par unité de la composition.

2. Composition selon la revendication 1, caractérisée par le fait que la proportion de cire dans le médicament revêtu de cire est de 0,05 à 5 parties en poids pour 1 partie en poids du médicament.

3. Composition selon l'une des revendications 1 et 2, caractérisée par le fait que la cire comprend de l'huile hydrogénée.

4. Procédé de préparation d'une composition solide de médicament en microdose choisi entre le fumarate de formotérol et la β-méthyldigoxine, caractérisé par le fait que l'on forme d'abord des particules de ce médicament revêtues de cire (1) en dispersant directement le médicament dans de la cire fondue ou (2) en dispersant le médicament en microdose dans de la cire dissoute dans un solvant et en éliminant le solvant et en formant de la poudre ou des granules de la dispersion ou du résidu obtenus, par congélation par pulvérisation, séchage par le vide ou séchage par pulvérisation.

5. Procédé selon la revendication 4, caractérisé par le fait que l'on revêt de la cire le médicament en microdose en même temps que de l'excipient.

6. Procédé selon l'une des revendications 4 et 5, caractérisé par le fait que la proportion de revêtement de cire est de 0,3 à 2 parties en poids pour 1 partie en poids du médicament.

8